# FASCICULE DE BREVET EUROPEEN

(11) **EP 0 586 392 B1**
(45) Date de publication et mention de la délivrance du brevet: **24.05.1995**
(21) Numéro de dépôt: 92907550.5
(22) Date de dépôt: 27.02.1992
(51) Int. Cl.: A61K 7/48, C11B 1/10, A61K 38/00

(54) **PROCEDE DE SEPARATION D'UN COMPOSE VEGETAL**
VERFAHREN ZUR TRENNUNG EINES PFLANZLICHEN PRÄPARATES
METHOD FOR SEPARATING A VEGETABLE COMPOUND

(30) Priorité: 27.05.1991 FR 9106336
(43) Date de publication de la demande: 16.03.1994
(73) Titulaire: LABORATOIRES INOCOSM, 92340 Bourg-la-Reine (FR)
(72) Inventeur: ROUSSET, Gérard, F-91320 Wissous (FR)
(74) Mandataire: Livet, Marie-José
(86) Numéro de dépôt international: FR9200182
(87) Numéro de publication internationale: WO9221321

(56) Documents cités:
- EP-A- 0 278 505
- WO-A-84/01710
- WO-A-86/01713
- WO-A-90/05521
- FR-A- 2 379 282
- FR-A- 2 400 552
- GB-A- 1 323 330
- PATENT ABSTRACTS OF JAPAN vol. 12, no. 37 (C-473)4 Février 1988
- AGRICULTURAL AND BIOLOGICAL CHEMISTRY. vol. 49, no. 12, 1985, TOKYO JP pages 3609 - 3611; MASAO OHNISHI ET AL.: 'Sphingolipid classes and their molecular species in wheat flour'
- LIPIDS vol. 19, no. 12, 1984, US pages 858 - 965; A.U.OSAGIE ET AL.: 'Lipid composition of millet (Pennisetum americanum) seeds'
- CEREAL CHEMISTRY vol. 51, no. 1, 1974, ST. PAUL, USA pages 17 - 33; M. J. L.LIN ET AL.: 'Hard red spring and durum wheat polar lipids. I. Isolation and quantitative determinations'
- WORLD PATENTS INDEX LATEST Section Ch, Week 8407, Derwent Publications Ltd.,London, GB; Class D, AN 84-039643
- JOURNAL OF CEREAL SCIENCE vol. 12, no. 1, 1990, pages 63 - 71; H.WIESER ET AL.:'Characterization of ethanol-extractable reduced subunits of glutenin separated by reversed-phase high-performance liquid chromatography'

## Description

La présente invention concerne un procédé de séparation d'un composé renfermant des glycolipides lysophospholipides sphingolipides et céramides d'origine végétale.

L'hydratation de la peau constitue l'un des problèmes majeurs posés aux spécialistes de la cosmétologie : il est en effet bien connu que l'eau constitue un élément vital de l'organisme humain ; sa concentration qui atteint 60 à 70 % au niveau du derme, chute à 12 % dans le stratum corneum, c'est-à-dire les dernières couches de l'épiderme.

Or, il est essentiel, pour conserver à la peau sa souplesse et son élasticité et empêcher la formation prématurée de rides ou de ridules de maintenir cette teneur en eau, en évitant toute déshydratation consécutive à des phénomènes de sudation ou d'évaporation par voie cutanée.

Pour remédier aux conséquences des phénomènes susmentionnés et retarder autant que possible le vieillissement de la peau, les spécialistes de la cosmétologie ont mis au point différentes compositions destinées à rétablir la teneur en eau des couches supérieures de l'épiderme soit en freinant leur déshydratation, soit en favorisant leur réhydratation.

Les premières de ces compositions, proposées vers le début des années 60, correspondaient à des crèmes à base de vaseline ou de cire d'abeille qui n'ont pas toujours été très bien accueillies par les consommateurs compte tenu de leur caractère gras et huileux.

Pour remédier à cet inconvénient, on a ensuite mis au point des compositions sous forme d'émulsions faites d'un mélange d'eau et d'huile présentant l'avantage d'être beaucoup plus agréables à utiliser.

A côté de ces compositions qui agissent principalement en apportant aux peaux sèches les lipides dont elles ont tendance à manquer, on a vu apparaître des produits hydratants plus sophistiqués comportant, en tant qu'agents actifs, des composants humectants ou hygroscopiques susceptibles, par affinité, de capter et de retenir l'eau environnante de façon à maintenir autour des cellules et à la surface de la peau une atmosphère humide. Parmi ces composants actifs, on peut noter la lanoline, les lécithines de soja, la glycérine, les mucopolysaccharides ou encore les dérivés d'allantoïne.

A côté de ces agents humectants ou hygroscopiques, les compositions hydratantes qui sont actuellement proposées aux consommateurs contiennent très souvent des produits dits 〈〈 natural moisturising factors 〉〉 qui sont normalement présents dans l'épiderme, et pour lesquels on a pu constater un déficit chez les sujets ayant une peau déshydratée ou à tendance sèche ; ces produits, dont on a pu vérifier l'intervention dans les mécanismes d'hydratation de la peau sont à titre d'exemple des acides aminés (sérine, lysine, tyrosine, ...), l'urée, le sodium, le potassium, l'acide pyrrolydone carboxylique...

Plus récemment encore, on a pu mettre en évidence le rôle dans les mécanismes d'hydratation de la peau des phospholipides, et surtout des céramides qui sont eux aussi normalement présents dans la couche cornée : on a en effet pu démontrer que ces produits agissent comme du ciment entre les briques des cellules et contribuent donc à une meilleure rétention d'eau et résistance de la peau aux agressions extérieures.

Il existe actuellement, sur le marché des cosmétiques, des compositions hydratantes (sous forme de crèmes, gels ou solutions) contenant des céramides associés à des agents de texture permettant de 〈〈 bétonner 〉〉 le tissu intercellulaire qui se sont révélées particulièrement efficaces et reçoivent par suite un accueil très favorable auprès de la clientèle.

Les céramides présents dans ces compositions présentent toutefois l'inconvénient d'être d'origine animale, et d'être extraits du cerveau ou de tissus nerveux animaux.

Cet inconvénient pourrait être de nature à freiner voire à stopper complètement le développement dans l'avenir des produits hydratants à base de céramides, étant donné que, pour des raisons écologiques, on cherche actuellement à protéger au maximum les animaux , et à éviter autant que possible de les utiliser au niveau du laboratoire : cette tendance actuelle, encouragée par les diverses ligues pour la protection des bêtes, fait que les produits d'origine animale sont de plus en plus mal acceptés.

Indépendamment de ces préoccupations d'ordre philosophique, la principale raison pour laquelle le développement des produits, notamment des produits cosmétiques provenant du règne animal, pourrait se trouver freiné dans l'avenir, est liée à l'apparition chez les ruminants de nouveaux virus du type prions se développant au niveau du cerveau et des tissus nerveux et qui sont notamment responsables de la maladie dite des 〈〈 vaches folles 〉〉. Ces virus semblent en effet actuellement muter, et il n'est pas exclu qu'ils puissent également se propager et se développer chez les humains, ce qui pourrait avoir des conséquences dramatiques.

Le risque susmentionné pourrait provoquer chez les chercheurs un revirement ayant pour conséquence une utilisation croissante du règne végétal ou des produits d'origine marine en remplacement de produits animaux, et ce, malgré des difficultés accrues liées au fait que les produits animaux se trouvent déjà dans un état partiellement ou totalement synthétisé tandis que l'utilisation de produits végétaux exige toujours des manipulations complémentaires pouvant s'avérer longues et coûteuses.

La présente invention, qui se place dans le contexte susmentionné a pour objet un procédé de séparation d'un composé renfermant des glycolipides lysophospholipides sphingolipides et céramides d'origine végétale, pouvant être ensuite utilisé pour la fabrication de compositions cosmétologiques hydratantes, et donc de nature à remédier aux inconvénients de la présence dans de telles compositions de céramides extraits de cerveaux ou de cellules nerveuses animales.

Il est à cet effet à noter qu'il est connu notamment par le document AGRICULTURAL AND BIOLOGICAL CHEMISTRY, vol. 49, n° 12, 1985, TOKYO JP, pages 3609-3611, que des glycosyl-céramides et des céramides sont présentes dans certains composés végétaux tels que la farine de blé.

Il est également à noter que l'on connaît :
- par le document WO-A-84 01 710 un produit cosmétique comprenant outre de la lysine et de l'acide gibberellique de la gliadine d'origine quelconque en tant que constituant actif annexe,
- par le document FR-A-2 379 282 un produit cosmétique et plus particulièrement un déodorant comprenant de l'hordénine, des amino acides, des acides gras organiques, des lipides et des glycides d'orge,
- par le document WO-A-86 01 713 un produit cosmétique renfermant des extraits de farine d'épis de maïs et d'autres résidus de la culture du maïs,
- par le document GB-A-1 323 330 un produit cosmétique obtenu à partir d'un matériau d'origine végétale ou animale renfermant des phospholipides,
- par le document EP-A-0 278 505 un produit cosmétique à usage capillaire renfermant en tant qu'agent actif des céramides combinés à des esters du cholestérol, et
- par le document WO-A-90 05 521 un produit cosmétique renfermant de la matière grasse d'extrait de farine de blé qui est obtenu à partir d'un constituant particulier de cette farine : le gluten.

Le procédé, conforme à l'invention, est caractérisé en ce que l'on chauffe une quantité donnée d'un alcool liquide, notamment de méthanol ou d'éthanol à une température de 50 à 80°C, on y introduit sous vive agitation un composé végétal tel que de la farine de céréales ou un extrait tel que du son ou des lipides extraits de céréales par des solvants chlorés tels que chlorure de méthylène ou chloroforme, on poursuit l'agitation pendant environ 30 minutes et on laisse reposer le mélange pendant environ 1 heure en maintenant la température , puis on le filtre à chaud de manière à séparer un filtrat d'un gâteau de filtration délipidé et on évapore l'alcool du filtrat de manière à obtenir un résidu contenant des glycolipides lysophospholipides sphingolipides et céramides d'origine végétale.

L'alcool utilisé peut être un alcool liquide quelconque, mais la législation propre au domaine de la cosmétologie impose d'utiliser un alcool dénaturé ; pour des raisons de prix de revient, il est avantageux de recycler l'alcool ayant déjà servi à effectuer une ou plusieurs extractions.

Le produit végétal le plus fréquemment utilisé conformément à l'invention est néanmoins la farine de blé entière et ce tant pour des raisons de prix de revient que de rendement.

La quantité de farine de blé entière devant être ajoutée correspond à 50 % en poids de la quantité d'alcool mise en oeuvre au départ.

Il est à noter que l'extraction à l'alcool chaud permet de solubiliser à la fois les triglycérides (les phospholipides, les glycolipides et les céramides et également de purifier les céramides car elles sont pratiquement les seules à précipiter à faible température dans l'alcool.

Selon une première variante de l'invention, on évapore l'alcool du filtrat en utilisant un ballon plongeant notamment dans un bain d'huile ; il s'agit là en quelque sorte d'un réacteur de type alambic. Il est ainsi possible de récupérer au fond du ballon un résidu pâteux homogène contenant 3 à 6 % de céramides.

La mise en oeuvre de ce procédé entraîne un travail relativement long et fastidieux qu'une seconde variante de l'invention, 〈〈 plus élégante 〉〉 permet d'éviter.

Selon cette variante, on refroidit le filtrat riche en lipides séparé du gâteau de filtration délipidé à une température de -20 à +4°C de manière à obtenir la précipitation sélective d'un mélange renfermant des céramides puis on filtre une seconde fois le produit en maintenant la température ainsi de récupérer un produit contenant des céramides et de l'alcool, on laisse ensuite revenir le produit à température ambiante de façon à séparer les céramides de l'alcool et on évapore l'alcool résiduel.

Il est conseillé de faire subir à la pâte lipidique renfermant des céramides un traitement complémentaire par exemple de lavage à l'acétone ou d'évaporation à l'étude pour retirer l'alcool restant : on peut ainsi recueillir en fin de processus une poudre lipidique contenant 80 à 90 % de céramides, 10 à 20 % de phospholipides et 10 à 20 % de glycolipides. Cette poudre peut être transférée telle quelle à un laboratoire dermatologique ou cosmétologique pour la fabrication d'une composition hydratante ou bien être séchée et pulvérisée de façon connue en elle-même de manière à obtenir 5 à 15 grammes de poudre par kilogramme de farine mise en oeuvre. Une telle poudre présente l'avantage d'être particulièrement stable, ce qui lui permet d'être conditionnée directement, notamment dans des sachets en aluminium, sans nécessiter l'adjonction d'agents de conservation ; elle peut également être mise en solution aqueuse ou huileuse.

L'étape suivante de ce procédé consiste à traiter le filtrat obtenu à l'issue de la seconde filtration. Celui-ci contient avantageusement 50 % de triglycérides, 25 % de phospholipides et 25 % de glycolipides.

Dans ce but on évapore l'alcool de façon connue en elle-même et on solubilise les triglycérides par l'acétone de façon à obtenir une poudre qui contient avantageusement 50 % de glycolipides, 25 % de phospholipides et 25 % de lysophospholipides. Les glycolipides peuvent être utilisés en dermatologie ou cosmétologie en tant qu'hydratants et les phospholipides et lysophospholipides peuvent être utilisés comme émulsifiants comme vecteurs de principes actifs et comme composants de liposomes, les lysophospholipides sont de meilleurs émulsifiants que les phospholipides.

Il est à noter que le blé est particulièrement riche en lysophospholipides.

L'invention se rapporte également à l'utilisation dans le domaine de la cosmétologie ou de la dermatologie du produit d'origine végétale renfermant un mélange lipidique contenant des glycolipides lysophospholipides sphingolipides et céramides d'origine végétale, tel qu'obtenu par la mise en oeuvre du procédé susmentionné.

Il est à noter que l'on peut envisager d'introduire un tel produit dans des liposomes.

Des produits cosmétiques d'origine végétale renfermant des sphingolipides avaient déjà été proposés par le document PATENT ABSTRACT OF JAPAN, vol. 12, n° 37 (C-473), 4 février 1988 ; ces produits cosmétiques ne renfermaient toutefois ni glycolipides, ni lisophospholipides, ni céramides.

Conformément à l'invention, on cherche également à 〈〈 rentabiliser 〉〉 le gâteau de filtration délipidé obtenu à l'issue de la première filtration pour la fabrication de différents produits cosmétiques ou dermatologiques.

L'une des possibilités offertes dans ce sens consiste à préparer de la gliadine végétale. La gliadine est une protéine végétale soluble dans les solutions hydroalcooliques, ce qui fait qu'elle est particulièrement intéressante en dermatologie ou cosmétologie.

A cet effet, et selon une autre caractéristique de l'invention, on ajoute à ce gâteau 20 à 40 % en poids d'eau déminéralisée, on agite la pâte ainsi obtenue pendant une heure à température ambiante et on la laisse décanter pendant une durée de trois jours, on récupère la fraction surnageante riche en gliadine végétale et on la soumet à une filtration sur papier filtre de manière à récupérer sur ce papier une pâte renfermant de la gliadine végétale pouvant être utilisée en cosmétologie.

Le produit renfermant de la gliadine végétale obtenu par la mise en oeuvre du procédé susmentionné peut être rajouté aux produits alcooliques pour empêcher l'irritation et éviter l'apparition sur le visage des utilisateurs de rougeurs caractéristiques. Il peut également être utilisé comme hydratant dans les milieux hydroalcooliques.

Après l'extraction de la gliadine, on peut utiliser le gâteau restant pour la fabrication d'un produit cosmétique riche en protéines natives du type de ceux connus et généralement commercialisés sous le nom de 〈〈 tenseurs 〉〉 et qui sont habituellement utilisés pour le nettoyage de la peau.

Selon une autre caractéristique de l'invention, pour fabriquer un tel tenseur, on récupère le gâteau de filtration délipidé susmentionné, on l'introduit en proportion de 50 % en poids dans un mélange d'eau déminéralisée, de chlorure de sodium à 0,9 à 3 % et le cas échéant d'agents de conservation, on agite la pâte ainsi obtenue pendant une heure et on la laisse décanter une douzaine d'heures à température ambiante, on récupère la fraction surnageante renfermant des protéines, on la chauffe à une température de 40 à 50°C, on la soumet à au moins une filtration sur papier filtre de manière à récupérer sur ce papier une pâte renfermant des protéines constituant le tenseur végétal.

Avant d'effectuer ce traitement, il est avantageux de faire subir au gâteau un lavage préalable à l'eau pure afin d'éviter autant que possible la présence d'alcool dans la suite du procédé.

En tant qu'agent de conservation, on peut utiliser un mélange de phénoxyéthanol, d'acide citrique et de parahydroxybenzoate de méthyl sodé, c'est-à-dire de trois agents de conservation agréés au niveau international ; il est à noter que la présence de ces conservateurs n'est pas nécessaire lorsque le tenseur végétal est destiné à être fortement dilué en vue de son utilisation. A titre d'exemple, on a pu obtenir des résultats intéressants en ajoutant à l'eau salée de départ environ 0,6 % de phénoxyéthanol, 0,6 % d'acide citrique et 0,4 % de parahydroxybenzoate de méthyl sodé.

Selon une autre caractéristique de l'invention, on soumet la fraction surnageante renfermant des protéines à une première filtration à une température de 40 à 50°C, on la laisse refroidir à l'air ambiant pendant une douzaine d'heures et on la soumet à une seconde filtration à une température de 20°C.

Il est avantageux d'ajouter au mélange, avant chauffage à 40 à 50°C, 1 % à 5 % de charbon actif (notamment le produit commercialisé par la Société MERCK sous l'appellation CLAROCARBONE) qui joue le rôle d'agent décolorant et désodorisant.

Le tenseur végétal obtenu par la mise en oeuvre du procédé susmentionné, peut être utilisé dans le domaine de la cosmétologie ou de la dermatologie à différentes dilutions.

Lorsqu'il est très dilué (0,2 / 0,3 % de protéine) il peut constituer un produit de nettoyage de la peau à haut pouvoir tolérant pouvant être utilisé sans nécessité de rinçage ; un tel produit est particulièrement intéressant pour le nettoyage de peaux souffrant d'acné, psoriasis, eczéma...

A une concentration voisine de 6 % en protéines, ce produit peut être utilisé pour la fabrication de crèmes de nature à 〈〈 tirer 〉〉 la peau ; il présente alors un pouvoir tenseur comparable à celui du sérum bovin ou de cheval.

A cette concentration, on obtient un produit comparable à ceux utilisés pour effacer les rides et donner un 〈〈 coup d'éclat 〉〉.

Il est également à noter que, à une concentration de l'ordre de 10 % en protéines, ce produit peut être mis en oeuvre dans des compositions destinées au raffermissement des seins.

En conséquence, l'invention permet également d'obtenir un tenseur végétal pouvant donner lieu à diverses applications, aussi bien sous forme de solution que de crème ou de gel.

## Revendications

1. Procédé de séparation d'un composé renfermant des glycolipides lysophospholipides sphingolipides et céramides d'origine végétale, caractérisé en ce que l'on chauffe une quantité donnée d'un alcool liquide, notamment de méthanol ou d'éthanol à une température de 50 à 80°C, on y introduit sous vive agitation un composé végétal tel que de la farine de céréales ou un extrait tel que du son ou des lipides extraits de céréales par des solvants chlorés tels que chlorure de méthylène ou chloroforme, on poursuit l'agitation pendant 30 minutes et on laisse reposer le mélange pendant 1 heure en maintenant la température, puis on le filtre à chaud de manière à séparer un filtrat d'un gâteau de filtration délipidé et on évapore l'alcool du filtrat de manière à obtenir un résidu lipidé contenant des glycolipides lysophospholipides sphingolipides et céramides d'origine végétale et pouvant être utilisé en cosmétologie ou en dermatologie.

2. Procédé selon la revendication 1, caractérisé en ce que l'on introduire dans l'alcool chaud 50 % en poids de farine de blé entière.

3. Procédé selon l'une quelconque des revendications 1 et 2, caractérisé en ce que l'on évapore l'alcool du filtrat en utilisant un ballon plongeant notamment dans un bain d'huile de manière à récupérer au fond du ballon un résidu pâteux homogène contenant 3 à 6 % de céramides.

4. Procédé selon l'une quelconque des revendications 1 et 2, caractérisé en ce que l'on refroidit le filtrat renfermant des en lipides séparé du gâteau de filtration délipidé à une température de -20 à +4°C de manière à obtenir la précipitation sélective d'un mélange renfermant des céramides puis on filtre une seconde fois le produit en maintenant la température afin de récupérer un produit renfermant des céramides, on laisse ensuite revenir le produit à température ambiante de façon à séparer les céramides de l'alcool et on évapore l'alcool résiduel et on recueille une poudre lipidique contenant 80 à 90 % de céramides.

5. Procédé selon la revendication 4, caractérisé en ce que l'on récupère le filtrat obtenu à l'issue de la seconde filtration qui contient 50 % de triglycérides, 25 % de phospholipides et 25 % de glycolipides, on évapore l'alcool de façon connue en elle-même et on solubilise les triglycérides par l'acétone de façon à obtenir une poudre qui contient avantageusement 50 % de glycolipides, 25 % de phospholipides et 25 % de lysophospholipides et peut être utilisé en dermatologie ou cosmétologie.

6. Procédé selon l'une quelconque des revendications 1 à 5, caractérisé en ce que l'on récupère le gâteau de filtration délipidé obtenu à l'issue de la première filtration, on ajoute à ce gâteau 20 à 40 % en poids d'eau déminéralisée, on agite la pâte ainsi obtenue pendant une heure à température ambiante et on la laisse décanter pendant une durée de trois jours, on récupère la fraction surnageante riche en gliadine végétale et on la soumet à une filtration sur papier filtre de manière à récupérer sur ce papier une pâte renfermant de la gliadine végétale pouvant être utilisée en cosmétologie.

7. Procédé selon la revendication 6, caractérisé en ce que l'on récupère le gâteau de filtration restant après l'extraction de la gliadine, on l'introduit en proportion de 50 % en poids dans un mélange d'eau déminéralisée, de chlorure de sodium à 0,9 à 3 % et le cas échéant d'agents de conservation, on agite la pâte ainsi obtenue pendant une heure et on la laisse décanter une douzaine d'heures à température ambiante, on récupère la fraction surnageante, on la chauffe à une température de 40 à 50°C, on la soumet à au moins une filtration sur papier filtre de manière à récupérer sur ce papier une pâte renfermant des protéines natives pouvant être utilisée en dermatologie ou en cosmétologie en tant que tenseur.

8. Procédé selon la revendication 7, caractérisé en ce que l'on soumet la fraction surnageante à une première filtration à une température de 40 à 50°C, on la laisse refroidir à l'air ambiant pendant une douzaine d'heures et on la soumet à une seconde filtration à une température de 20°C.

## Claims

1. Method for separating a compound containing glycolipids, lysophospholipids, sphingolipids and ceramides of plant origin, characterised in that a given amount of a liquid alcohol, in particular methanol or ethanol, is heated to a temperature of from 50 to 80°C, a plant compound such as cereal flour or an extract such as bran or lipids extracted from cereals by chlorinated solvents such as methylene chloride or chloroform is introduced into the alcohol under intensive stirring, stirring is continued for 30 minutes, and the mixture is left to stand for one hour whilst the temperature is maintained, then it is hot-filtered so as to separate a filtrate from a delipidised filter cake and the alcohol is evaporated from the filtrate in such a manner that a lipid residue which contains glycolipids, lysophospholipids, sphingolipids and ceramides of plant origin and which can be used in cosmetology or dermatology is obtained.

2. Method according to Claim 1, characterised in that 50% by weight of whole wheat flour is introduced into the hot alcohol.

3. Method according to either of Claims 1 and 2, characterised in that the alcohol is evaporated from the filtrate using a flask immersed in particular in an oil bath in such a manner that a homogeneous pasty residue which contains from 3 to 6% of ceramides is recovered at the bottom of the flask.

4. Method according to either of Claims 1 and 2, characterised in that the filtrate containing the lipids and separated from the delipidised filter cake is cooled to a temperature of from -20 to +4°C in such a manner that a ceramide-containing mixture is precipitated selectively, the product is then filtered for a second time whilst the temperature is maintained in order to recover a ceramide-containing product, the product is then left to return to ambient temperature so as to separate the ceramides from the alcohol, the residual alcohol is evaporated, and a lipid powder containing from 80 to 90% of ceramides is recovered.

5. Method according to Claim 4, characterised in that the filtrate which is obtained at the end of the second filtration and which contains 50% of triglycerides, 25% of phospholipids and 25% of glycolipids is recovered, the alcohol is evaporated in a manner known per se, and the triglycerides are solubilised with acetone in such a manner that a powder advantageously containing 50% of glycolipids, 25% of phospholipids and 25% of lysophospholipids and which can be used in dermatology or cosmetology is obtained.

6. Method according to any one of Claims 1 to 5, characterised in that the delipidised filter cake obtained at the end of the first filtration is recovered, from 20 to 40% by weight of demineralised water is added to this cake, the resultant paste is stirred for one hour at ambient temperature, and it is left to settle for three days, the supernatant fraction which is rich in plant gliadin is recovered and is subjected to filtration on a filter paper in such a manner that a paste which contains plant gliadin and which can be used in cosmetology is recovered on this paper.

7. Method according to Claim 6, characterised in that the filter cake remaining after the extraction of the gliadin is recovered, it is introduced in a proportion of 50% by weight into a mixture of demineralised water, from 0.9 to 3% sodium chloride and, optionally, preservatives, the resultant paste is stirred for one hour and it is left to settle for approximately twelve hours at ambient temperature, the supernatant fraction is recovered, it is heated to a temperature of from 40 to 50°C, and it is subjected to at least one filtration on a filter paper in such a manner that a paste which contains native proteins and which can be used as a tensing agent in dermatology or cosmetology is recovered on this paper.

8. Method according to Claim 7, characterised in that the supernatant fraction is subjected to a first filtration at a temperature of from 40 to 50°C, it is left to cool in the ambient air for approximately twelve hours, and it is subjected to a second filtration at a temperature of 20°C.

## Patentansprüche

1. Verfahren für die Zerlegung einer Verbindung, welche Glykolipide, Lysophospholipide, Sphingolipide und Keramide pflanzlichen Ursprungs enthält, dadurch gekennzeichnet, daß eine gegebene Menge eines flüssigen Alkohols, insbesondere Methanol oder Äthanol, auf eine Temperatur von 50 bis 80°C erwärmt wird, daß dazu unter kräftigem Rühren eine pflanzliche Verbindung zugegeben wird, wie etwa Getreidemehl oder ein Extrakt, wie etwa von Kleie, oder Lipide, die mit Hilfe chlorhaltiger Lösungsmittel, wie Methylenchlorid oder Chloroform, aus Getreide extrahiert wurden, daß man das Rühren 30 Minuten fortsetzt und dann die Mischung eine Stunde lang bei Aufrechterhaltung der Temperatur ruhen läßt, daß sie dann warm gefiltert wird, in der Weise, daß ein Filtrat von einem entlipidierten Filterkuchen getrennt wird, und daß der Alkohol aus dem Filtrat verdampft wird, so daß man einen Lipide-Rückstand erhält, welcher Glykolipide, Lysophospholipide, Sphingolipide und Keramide pflanzlichen Ursprungs enthält und in der Kosmetikkunde oder in der Dermatologie verwendet werden kann.

2. Verfahren nach Anspruch 1, dadurch gekennzeichnet, daß man zu dem heißen Alkohol 50 Gewichtsprozent Weizenvollkornmehl zugibt.

3. Verfahren nach einem der Ansprüche 1 und 2, dadurch gekennzeichnet, daß der Alkohol des Filtrats verdampft wird, indem ein Kolben benutzt wird, der speziell in ein Ölbad eingetaucht wird, derart, daß am Boden des Kolbens ein homogener teigiger Rückstand erhalten wird, der 3 bis 6% Keramide enthält.

4. Verfahren nach einem der Ansprüche 1 und 2, dadurch gekennzeichnet, daß das Lipide enthaltende, von dem entlipidierten Filterkuchen getrennte Filtrat auf eine Temperatur zwischen -20 und +4°C abgekühlt wird, so daß das selektive Ausfällen einer Keramide enthaltenden Mischung bewirkt wird, wonach das Produkt unter Beibehaltung der Temperatur ein zweites Mal gefiltert wird, um ein Produkt zu erhalten, das Keramide enthält, und daß man das Produkt anschließend wieder Raumtemperatur annehmen läßt, so daß die Keramide von dem Alkohol getrennt werden, und daß der restliche Alkohol verdampft wird und ein Lipidpulver gewonnen wird, welches 80 bis 90% Keramide enthält.

5. Verfahren nach Anspruch 4, dadurch gekennzeichnet, daß das im Ergebnis der zweiten Filterung erhaltene Filtrat gewonnen wird, welches 50% Triglyzeride, 25% Phospholipide und 25% Glykolipide enthält, daß der Alkohol in an sich bekannter Weise verdampft wird und daß die Triglyzeride mittels Azeton löslich gemacht werden, derart, daß ein Pulver erhalten wird, das vorteilhafterweise 50% Glykolipide, 25% Phospholipide und 25% Lysophospholipide enthält und in der Dermatologie oder Kosmetikkunde verwendet werden kann.

6. Verfahren nach einem der Ansprüche 1 bis 5, dadurch gekennzeichnet, daß der im Ergebnis der ersten Filterung erhaltene entlipidierte Filterkuchen gewonnen wird, daß zu diesem Filterkuchen 20 bis 40 Gewichtsprozent entsalztes Wasser zugegeben werden, daß der so erhaltene Brei eine Stunde lang bei Raumtemperatur gerührt wird, und daß man ihn drei Tage lang sich absetzen läßt, daß der obenauf schwimmende, an pflanzlichem Glyadin reiche Überstand gewonnen wird und einer Filterung durch Filterpapier unterzogen wird, derart, daß auf diesem Filterpapier ein Brei erhalten wird, welcher pflanzlichen Glyadin enthält, das in der Kosmetikkunde verwendet werden kann.

7. Verfahren nach Anspruch 6, dadurch gekennzeichnet, daß der nach der Extraktion des Glyadins Verbleibende Filterkuchen gewonnen und in einem Verhältnis von 50 Gewichtsprozent einer Mischung aus entgalztem Wasser, 0,9 bis 3% Natriumchlorid und gegebenenfalls Konservierungsmitteln zugegeben wird, daß der so erhaltene Brei eine Stunde lang gerührt wird und daß man, ihn etwa zwölf Stunden lang bei Umgebungstemperatur sich abgetzen läßt, daß der obenauf schwimmende Überstand gewonnen wird, daß er auf eine Temperatur von 40 bis 50°C erwärmt wird, und daß er mindestens einer Filterung durch Filterpapier unterzogen wird, derart, daß auf diesem Filterpapier ein Brei erhalten wird, welcher natürliche Proteine enthält, die in der Dermatologie oder in der Kosmetikkunde als Tensor verwendet werden können.

8. Verfahren nach Anspruch 7, dadurch gekennzeichnet, daß der obenauf schwimmende Überstand einer ersten Filterung bei einer Temperatur von 40 bis 50°C unterzogen wird, daß man ihn etwa 12 Stunden lang an der umgebenden Luft abkühlen läßt und daß er einer zweiten Filterung bei einer Temperatur von 20°C unterzogen wird.
